(19) Europäisches Patentamt
European Patent Office
Office européen des brevets

(11) **EP 4 140 578 A1**

(12) **EUROPEAN PATENT APPLICATION**
published in accordance with Art. 153(4) EPC

(43) Date of publication:
**01.03.2023 Bulletin 2023/09**

(21) Application number: **20932812.9**

(22) Date of filing: **23.04.2020**

(51) International Patent Classification (IPC):
**B01J 23/648** (2006.01)  **B01J 23/652** (2006.01)
**B01J 27/185** (2006.01)  **C01B 3/26** (2006.01)
**C07C 5/367** (2006.01)

(52) Cooperative Patent Classification (CPC):
**B01J 23/648; B01J 23/652; B01J 27/185;
C01B 3/26; C07C 5/367;** Y02P 20/52

(86) International application number:
**PCT/JP2020/017564**

(87) International publication number:
**WO 2021/214955 (28.10.2021 Gazette 2021/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Chiyoda Corporation
Kanagawa 220-8765 (JP)**

(72) Inventors:
• **OKADA Yoshimi
Yokohama-shi, Kanagawa 220-8765 (JP)**

• **IMAGAWA Kenichi
Yokohama-shi, Kanagawa 220-8765 (JP)**
• **SAITO Masashi
Yokohama-shi, Kanagawa 220-8765 (JP)**
• **FUKUDOME Kenta
Yokohama-shi, Kanagawa 220-8765 (JP)**
• **KOBAYASHI Haruto
Yokohama-shi, Kanagawa 220-8765 (JP)**

(74) Representative: **Dehns
St. Bride's House
10 Salisbury Square
London EC4Y 8JD (GB)**

(54) **EGGSHELL TYPE PLATINUM-LOADED ALUMINA CATALYST, METHOD FOR PRODUCING SAME, AND USE OF SAME**

(57)    To provide an egg shell-type platinum-loaded alumina catalyst demonstrating excellent performance in terms of catalyst life, an egg shell-type platinum-loaded alumina catalyst includes: an alumina carrier; platinum dispersed and loaded on an outer shell of the alumina carrier; and one or more second components selected from the group consisting of vanadium, chromium, molybdenum, and phosphorus. Preferably, the content of platinum is 0.05 to 5.0 wt% calculated as elemental platinum. The content of each second component preferably is 0.1 to 5.0 wt% calculated as each element. The alumina carrier has a surface area of 150 $m^2$/g or more, a pore volume of 0.40 $cm^3$/g or more, and an average pore diameter of 40 to 300 Å, with pores having a pore diameter in a range of ± 30 Å from the average pore diameter occupying 60 % or more of a total pore volume.

**Fig.1**

(A)          (B)

**Description**

TECHNICAL FIELD

The present invention relates to a metal catalyst for use in processes using catalyst, such as chemical product production, hydrogen production, fine chemical production, environment cleaning such as exhaust gas treatment, etc., and relates to an egg shell-type platinum-loaded alumina catalyst in which platinum is loaded on an alumina carrier, a method of producing the same, and a method of using the same.

BACKGROUND ART

A platinum-loaded alumina catalyst in which platinum or the like is loaded on an alumina carrier is industrially used in a very wide range of fields, such as production of fuel, petrochemical products, and fine chemicals such as medicine or environmental clean-up such as cleaning automobile exhaust gas, through dehydrogenation reaction, hydrogenation reaction, and reforming reaction of various compounds, such as dehydrogenation reaction of dehydrogenating hydrogenated aromatics such as methylcyclohexane, cyclohexane, decalin, and dibenzyltoluene, for example, into the corresponding aromatics and hydrogen.

Generally, such platinum-loaded alumina catalyst is manufactured as follows: a porous alumina carrier made of a metal oxide of alumina is prepared; the obtained porous alumina carrier is impregnated with a solution of a catalyst metal compound, such as a chloroplatinic acid aqueous solution, a platinum ammonium chloride aqueous solution, and a solution of an organoplatinum compound such as platinum acetylacetonate; the resultant is dried to form a dried matter on which the catalyst metal compound is loaded; the dried matter is calcined, e.g., at 350 to 800 °C for 0.5 to 24 hours to form a calcined matter on which the catalyst metal compound is loaded; and, as required, the obtained calcined matter on which the catalyst metal compound is loaded is subjected to hydrogen reduction, e.g., at 250 to 800 °C for 0.5 to 24 hours.

Regarding the platinum-loaded alumina catalyst manufactured by such a method, it is known that since the platinum atom with the atomic weight 195 has a large mass and the adsorbability of a platinum compound used as a platinum source to the catalyst carrier is high, the platinum compound is adsorbed by and fixed to the outer shell part of the alumina carrier before the platinum compound is dispersed to the inside of the alumina carrier, which forms a so-called egg shell-type platinum-loaded catalyst in which, when the dispersion state of platinum metal is observed in the catalyst cross section, the platinum metal is loaded only on the outer shell part of the catalyst and no platinum metal is loaded inside the carrier.

In the catalytic reaction in which the diffusion resistance inside the catalyst particles is high due to reasons such as that raw material molecules are large, the reaction occurs preferentially in the outer shell of the catalyst particles even if platinum as the active metal is loaded to the inside of the catalyst because the speed of diffusion of the raw material molecules to the inside of the catalyst is slow and the reaction does not progress sufficiently. In such a reaction, the egg shell-type catalyst in which the active metal exists in only the outer shell part of the catalyst is advantageous. However, when a certain amount of active metal is loaded only on the outer shell of the catalyst particles, the density of the active metal particles increases, which may lead to problems that the active metal particles cannot be sufficiently dispersed, catalyst deterioration due to sintering or coking is likely to occur, etc. From such a viewpoint, catalysts in which the dispersity of platinum metal when platinum metal is loaded only on the outer shell part of the catalyst is improved are being developed. Patent Document 1 discloses an egg shell-type platinum-loaded alumina catalyst in which the pore size of the platinum-loaded alumina catalyst is substantially uniform to such a degree that the diffusion resistance does not become large and the dispersion of platinum is good. Also, catalysts in which platinum metal is dispersed well over the entire cross section of the catalyst so that the surface area of the carrier is fully utilized in a reaction not affected by the diffusion resistance are being developed, and Patent Document 2 discloses such a uniform-type platinum-loaded alumina catalyst.

In the catalytic reaction in which the diffusion resistance inside the catalyst particles is high due to reasons such as that raw material molecules are large, the reaction occurs preferentially in the outer shell of the catalyst particles even if platinum as the active metal is loaded to the inside of the catalyst because the speed of diffusion of the raw material molecules to the inside of the catalyst is slow and the reaction does not progress sufficiently. In such a reaction, the egg shell-type catalyst in which the active metal exists in only the outer shell part of the catalyst is advantageous. However, when a certain amount of active metal is loaded only on the outer shell of the catalyst particles, the density of the active metal particles increases, which may lead to problems that the active metal particles cannot be sufficiently dispersed, catalyst deterioration due to sintering or coking is likely to occur, etc. From such a viewpoint, catalysts in which the dispersity of platinum metal when platinum metal is loaded only on the outer shell part of the catalyst is improved are being developed. Patent Document 1 discloses a platinum-loaded alumina catalyst in which the pore size of the platinum-loaded alumina catalyst is substantially uniform to such a degree that the diffusion resistance does not become large and the dispersion of platinum is good. Also, catalysts in which platinum metal is dispersed well over the entire cross section of the catalyst so that the surface area of the carrier is fully utilized in a reaction not affected by the diffusion resistance are being developed, and Patent Document 2 discloses such a platinum-loaded alumina catalyst.

The platinum-loaded alumina catalyst has been used in the catalytic process of a wide range of fields from old times, but in recent years is used in an organic chemical hydride method which is one method for hydrogen energy carrier and is attracting attention as storage and transportation technology for hydrogen energy. Development of platinum-loaded alumina catalyst having higher performance compared to the conventional platinum-loaded alumina catalyst is being in progress, and Patent Document 1 and Patent Document 2 disclose, as a use of the platinum-loaded alumina catalyst, use in the dehydrogenation reaction necessary in the organic chemical hydride method. The organic chemical hydride method is a method for "storing" and "transporting" hydrogen in the form of organic chemical hydride compounds (hydrogenated organic compounds) in which hydrogen is taken in the molecular structure of chemical products by chemical reaction. The organic chemical hydride method is a method that has been proposed since 1980s. However, the life of dehydrogenation catalyst for producing hydrogen from the organic chemical hydride compounds in which hydrogen is retained is very short, whereby industrial implementation thereof is difficult and the method has not been put into practice. The key to technological development is development of a novel dehydrogenation catalyst having sufficient performance such as a catalyst life that allows for industrial use. Currently, due to application of a platinum-loaded alumina catalyst having high performance as mentioned above, the development of a hydrogen energy carrier system based on the organic chemical hydride method has reached a point where demonstration of large-scale international hydrogen transportation is executed, and the development has technologically progressed to a stage where commercialization is possible. Non-Patent Documents 3 and 4 disclose details of such development of the organic chemical hydride method.

Japan has adopted a policy to promote practical implementation and dissemination of hydrogen energy as a national policy from the Fourth Strategic Energy Plan after the Great East Japan Earthquake, and following formulation of the Hydrogen Fuel Cell Technology Roadmap, the Hydrogen Basic Strategy was approved by the cabinet in 2017. The aforementioned organic chemical hydride method can provide a hydrogen energy carrier for "storing" and "transporting" hydrogen energy in a large scale, and the practical implementation thereof is incorporated in the Hydrogen Basic Strategy, which sets 30 ¥/Nm$^3$ as a target hydrogen supply price by 2030, and 20 ¥/Nm$^3$ by 2050. This requires cost reduction by continuous improvement technology development, and improvement of the catalyst performance, particularly the catalyst life, significantly affects the cost reduction. The development has progressed such that of the catalyst performance, the conversion rate, the selectivity, and the yield, which is a product of the conversion rate and the selectivity, have been improved to a relatively high level, and the development is now in a stage where improvement of the catalyst life, which determines how long the performance can be maintained, contributes to the cost reduction. Further, in the industrial catalyst production process, simplification of the procedure and process due to non-use of sulfur also can be an elemental technology that contributes to cost reduction.

PRIOR ART DOCUMENT(S)

PATENT DOCUMENT(S)

Patent Document 1: JP4652695B2
Patent Document 2: JP4142733B2
Non-Patent Document 3: OKADA Yoshimi, Energy/Natural Resources, Vol.33, No.3, 168 (2018)
Non-Patent Document 4: OKADA Yoshimi, Bulletin of The High Pressure Gas Safety Institute of TOKYO, August and September 2019
Non-Patent Document 5: Agency for Natural Resources and Energy, Hydrogen Basic Strategy (December 2017)

SUMMARY OF THE INVENTION

TASK TO BE ACCOMPLISHED BY THE INVENTION

The uniform-type platinum-loaded alumina catalyst of Patent Document 2 has a relatively long catalyst life and is practically useful, but further improvement is desired. Since the dehydrogenation reaction in the organic chemical hydride method is an endothermic reaction and an equilibrium reaction in which the number of molecules increases with the reaction, the efficiency is better under the reaction conditions at higher temperature and lower pressure. On the other hand, since the produced hydrogen may be preferred to be at high pressure depending on usage, a catalyst that can be used at higher reaction temperature may be demanded. However, under the reaction conditions at high temperature, side reaction tends to occur, and therefore, it is necessary to improve the selectivity of the catalyst. Also, under the reaction conditions at high temperature, carbon tends to be produced on the catalyst and the catalyst tends to deteriorate easily. Also, as mentioned above, the dehydrogenation reaction should be preferably carried out at low temperature. Therefore, catalysts with low pressure loss are required and catalysts with large particle diameter are advantageous. As the particle diameter of the catalyst increases, the gas diffusion to the inside of the catalyst reduces, and therefore, the egg shell-type catalyst is advantageous when the efficiency of the platinum used in the catalyst is taken into account.

Under the above background, the present inventors carried out extensive research on the performance improvement of the platinum-loaded alumina catalyst and, as a result, found that in the egg shell-type platinum-loaded alumina catalyst, addition of a second component other than alkali metal can improve the catalyst life.

Accordingly, an object of the present invention is to improve catalytic activity, selectivity, and particularly catalyst life in an egg shell-type platinum-loaded alumina catalyst using sulfur, which is advantageous when the diffusion rate of the raw material in the catalyst pores is low.

Also, another object of the present invention is to provide a method of such an egg shell-type platinum-loaded alumina catalyst demonstrating excellent performance in terms of catalytic activity, selectivity, and particularly catalyst life, and a method of using the same.

MEANS TO ACCOMPLISH THE TASK

One aspect of the present invention is an egg shell-type platinum-loaded alumina catalyst, comprising: an alumina carrier; platinum dispersed and loaded on an outer shell of the alumina carrier; and one or more second components selected from the group consisting of vanadium, chromium, molybdenum, and phosphorus. According to this aspect, the catalyst life can be improved.

Preferably, a content of the platinum is 0.05 to 5.0 wt% calculated as elemental platinum. Also preferably, a content of the second component is 0.1 to 5.0 wt% calculated as each element. The alumina carrier preferably has a surface area of 150 $m^2/g$ or more, a pore volume of 0.40 $cm^3/g$ or more, and an average pore diameter of 40 to 300 Å, with pores having a pore diameter in a range of $\pm$ 30 Å from the average pore diameter occupying 60 % or more of a total pore volume.

Another aspect of the present invention is a method of producing an egg shell-type platinum-loaded alumina catalyst, comprising: a step of impregnating an alumina carrier with an aqueous solution of compound containing one or more components selected from the group consisting of vanadium, chromium, molybdenum, and phosphorus, and thereafter performing drying and calcination; a step of loading platinum on the alumina carrier after calcination in an egg shell form; and a step of reducing the alumina carrier on which platinum is loaded in a hydrogen atmosphere.

Yet another aspect of the present invention provides a method of dehydrogenating a hydrogenated aromatic, comprising dehydrogenating a hydrogenated aromatic by using the egg shell-type platinum-loaded alumina catalyst. Preferably, the hydrogenated aromatic is one member or a mixture of two or more members selected from the group consisting of a hydride of monocyclic aromatic, a hydride of bicyclic aromatic, and a hydride of compound having 3 or more aromatic rings. Also preferably, the hydrogenated aromatic is one member or a mixture of two or more members selected from the group consisting of methylcyclohexane, cyclohexane, dimethylcyclohexane, tetralin, decalin, methyldecalin, biphenyl, diphenylmethyl, dibenzotriol, and tetradecahydroanthracene.

As described above, the uniform-type catalyst is effective when the raw material is sufficiently diffused to the inside of the catalyst, and the egg shell-type catalyst is effective when the diffusion to the inside of the catalyst is limited and is not performed sufficiently, and therefore, it is preferred to use these two types of catalysts properly depending on the state of diffusion in the reaction field. Also, even with the same reaction, the state of diffusion of the raw material to the inside of the catalyst may differ depending on the position in the reactor, and near the exit where the reaction has progressed, the raw material concentration becomes low, and the diffusion to the inside of the catalyst may be limited. In such a case, it is preferred to use both of the uniform-type and egg shell-type catalysts in the reactor.

In general, the extent of diffusion of the raw material to the inside of the catalyst is represented by a catalyst effectiveness factor, and the catalyst effectiveness factor can be controlled by changing the size and shape of the catalyst pellet. Thus, for both of the uniform-type and egg shell-type catalysts, it is possible to produce platinum-alumina catalysts having various catalyst effectiveness factors by changing the size and shape of the catalyst pellet.

In the egg shell-type platinum-loaded alumina catalyst of the present invention, it is preferred that the alumina carrier on which platinum and the second component exist has pore sizes controlled as uniformly as possible. Specifically, a porous metal oxide in which a sulfur-containing porous metal oxide has a surface area of 150 $m^2/g$ or more, a pore volume of 0.4 $cm^3/g$ or more, and an average pore diameter of 40 to 300 Å, with pores having a pore diameter in a range of $\pm$ 30 Å from the average pore diameter occupying 60 % or more of a total pore volume is preferred. As a result of that the pore sizes are made uniform as this, the alumina carrier has a uniform pore size over the entirety of the powder and molded body thereof.

Patent Document 1 discloses a platinum-loaded alumina catalyst in which platinum is loaded on a porous $\gamma$-

alumina carrier having a surface area of 150 m$^2$/g or more, a pore volume of 0.55 cm$^3$/g or more, and an average pore diameter of 90 to 300 Å, with pores having a pore diameter of 90 to 300 Å occupying 60 % or more of a total pore volume. This catalyst is a general egg shell-type platinum-loaded alumina catalyst as described above, and Patent Document 1 also discloses a catalyst to which alkali metal is added as means to improve the catalyst life. When using a catalyst with alumina as a carrier, merely inhibiting the decomposition reaction that occurs on the platinum particles is not enough, and it is also necessary to inhibit the decomposition reaction that occurs on the acid sites of the alumina. Therefore, in many cases, the decomposition reaction occurring on the alumina surface is inhibited by masking these acid sites using alkali metals such as potassium and lithium. From this viewpoint, Patent Document 1 discloses that in the egg shell-type platinum-loaded catalyst, loading of platinum with high dispersity inhibits the decomposition on platinum, and addition of alkali to mask the acid sites on alumina has a remarkable effect on the catalyst life improvement.

On the other hand, Patent Document 2 discloses a uniform-type platinum-loaded alumina catalyst in which sulfur is contained in the alumina carrier so that the dispersion mode of the loaded platinum becomes a uniform-type and the decomposition reaction is suppressed to improve the catalyst life. At the time when the invention of Patent Document 2 was made, it was considered that in the case where the alumina carrier containing sulfur or a sulfur compound is used, even when the acid sites are not masked with alkali metal thereon, the decomposition reaction inhibitory effect is equivalent to or higher than the inhibitory effect when the acid sites are masked with alkali metal. Although the detailed mechanism is not elucidated, it was considered that elemental sulfur forms a complex oxide with alumina, thereby altering the configuration of the acid sites, which remain in the case of using alumina alone, to a different configuration. In that case, the form obtained when elemental sulfur forms a complex oxide with alumina was generally considered to be in a sulfate group form.

In the production of the egg shell-type platinum-loaded alumina catalyst according to the present invention, additional inclusion of the second component addition process can be a cause of cost increase, but the cost reduction effect brought by the extension of the catalyst life according to the present invention is much greater than the increase in the catalyst production cost, and it is possible to extend the replacement life of the reactor the dehydrogenation catalyst from the conventional 1 to 2 years to 3 to 4 years depending on how the catalyst is used.

The present catalyst is typically used as a dehydrogenation catalyst, in which the catalyst fills the catalytic reaction tubes of a heat exchange-type reactor. As with general heat exchanges, the number of the catalytic reaction tubes may be thousands in a large reactor. When the platinum-loaded alumina catalyst used in such a reactor reaches a catalyst life, at which time the performance is lowered to result in a certain yield, the catalyst is removed to be replaced with new catalyst. Platinum is recovered from the removed waste catalyst and is recycled for use in the production of the catalyst for next replacement. The removing work may take several days, and the charging of the new catalyst may require more working days, and therefore, the catalyst replacement requires about two weeks. The production stops during that time, and therefore, the reduction of replacement frequency significantly contributes to the cost reduction. Namely, while the life of the catalysts disclosed by Patent Document 1 and Patent Document 2 is 1 to 2 years, the life of the alkali-added uniform-type platinum-loaded alumina catalyst according to the present invention is 4 years, and this makes it possible to reduce the catalyst replacement frequency to half or less.

EFFECT OF THE INVENTION

The egg shell-type platinum-loaded alumina catalyst of the present invention has higher catalyst performance, particularly with respect to the catalyst life, compared to the conventional egg shell-type platinum-loaded alumina catalyst. Also, these catalysts can be mass-produced easily by existing catalyst production facilities according to the manufacturing method of the present invention. These catalysts not only can be used as an alternative to the existing platinum-loaded alumina catalyst, but also can be favorably used as a dehydrogenation catalyst for methylcyclohexane or the like in the organic chemical hydride method, which is one of the hydrogen storage and transportation technologies.

BRIEF DESCRIPTION OF THE DRAWINGS

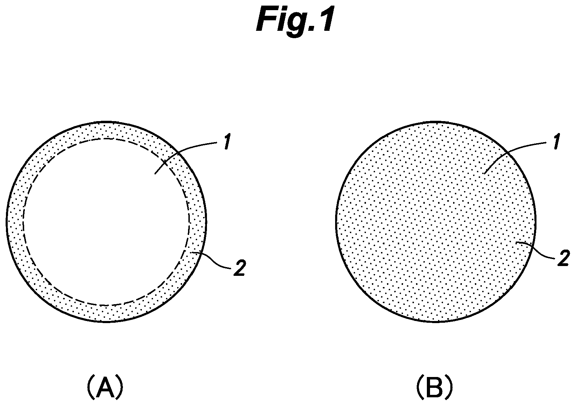

Figure 1 is an explanatory diagram showing (A) an egg shell-type metal-loaded catalyst and (B) a uniform-type metal-loaded catalyst.

MODE(S) FOR CARRYING OUT THE INVENTION

First, an egg shell-type metal-loaded catalyst and a uniform-type metal-loaded catalyst of the present invention will be described with reference to Figure 1. The egg shell-type metal-loaded catalyst refers to a state where a metal member to be loaded is dispersed and loaded only on the outer shell part of the cross section of a molded catalyst. Namely, a metal loading part 2 on which the metal member is loaded is formed in the outer shell part of a porous carrier 1. The uniform-type metal-loaded catalyst refers to a state where a metal member is dispersed over the entire cross section of the catalyst and the metal loading part 2 on which the metal member is loaded is formed over the entire inside of a molded body of the porous carrier 1.

The uniform-type platinum-loaded alumina catalyst includes an alumina carrier, sulfur or a sulfur compound dispersed over the entire cross section of the alumina carrier, platinum dispersed and loaded over the entire cross section of the alumina carrier, and one or more alkali metals selected from the group consisting of sodium, potassium, and calcium.

Next, the alumina carrier used in the present invention will be described. The alumina carrier has a surface area of 150 $m^2$/g or more, a pore volume of 0.40 $cm^3$/g or more, and an average pore diameter of 40 to 300 Å, with pores having a pore diameter in a range of $\pm$ 30 Å from the average pore diameter occupying 60 % or more of a total pore volume.

The alumina carrier preferably is a porous $\gamma$-alumina carrier. As disclosed in JPH6-72005B2, for example, the alumina carrier preferably is a porous $\gamma$-alumina carrier obtained by washing by filtration a slurry of aluminum hydroxide generated by neutralizing aluminum salt, dehydrating and drying the obtained alumina hydrogel, and then calcining the resultant at 400 to 800 °C for about 1 to 6 hours. More preferably, the alumina carrier is a porous $\gamma$-alumina carrier obtained through a pH swing process in which the pH of alumina hydrogel is alternately fluctuated between a pH range of the dissolution of alumina hydrogel and a pH range of the precipitation of boehmite gel and simultaneously an alumina hydrogel forming substance is added for growing crystals of the alumina hydrogel when the pH is fluctuated from at least either one of the pH ranges to the other one of the pH ranges. The porous $\gamma$-alumina carrier obtained through the pH swing process is excellent in the uniformity of pore distribution, and excellent in that the physical properties of each pellet are stable because there is less variation in the physical properties also in the alumina carrier pellet after the formation of the carrier.

In the present invention, the amount of addition of the second component loaded on the aforementioned alumina carrier is 0.1 to 5.0 wt%, preferably 0.3 to 3.0 wt%, and more preferably 0.5 to 2.0 wt%. If the loading amount of the second component is less than 0.1 wt%, there is a problem that the catalyst life is short and the effect is insignificant, while if the loading amount of the second component is greater than 5.0 wt%, there arises a problem that the activity becomes lower and the catalyst life becomes short.

The compound of the second component used when adding the second component to the aforementioned alumina carrier may be, for example, a chloride, bromide, iodide, nitrate, sulfate, acetate, propionic acid, and the like of the second component element, which preferably is water soluble and/or soluble to an organic solvent such as acetone. Such a compound may be, for example, vanadium chloride, vanadium bromide, vanadium iodide, ammonium metavanadate, vanadyl chloride, vanadyl sulfate, vanadium oxide acetyl acetate, chromium chloride, chromium bromide, chromium iodide, chromium nitrate, chromium sulfate, chromium acetate, chromium propionate, molybdenum chloride, molybdenum bromide, molybdenum iodide, molybdates, phosphoric acid, inorganic phosphate such as ammonium dihydrogen phosphate, organophosphate compound, or the like.

Also, when the second component element is added to the egg shell-type platinum-loaded alumina catalyst, it is desirable that the egg shell-type platinum-loaded alumina catalyst is impregnated with a solution of a second component element compound, thereafter dried under a drying condition at room temperature to 200 °C for 0.5 to 48 hours, preferably at 50 to 150 °C for 0.5 to 24 hours, and more preferably at 80 to 120 °C for 0.5 to 5 hours, and then calcined at 350 to 600 °C for 0.5 to 48 hours, more preferably at 350 to 450 °C for 0.5 to 5 hours.

In the present invention, the amount of platinum to be loaded on the aforementioned alumina carrier on which the second component is loaded is 0.05 to 5.0 wt%, preferably 0.1 to 3.0 wt%, calculated as elemental platinum. When the loading amount of platinum is less than 0.05 wt%, there is a problem that the activity is low, while when the loading amount of platinum exceeds 5.0 wt%, there are problems that the particle diameter of platinum increases, the selectivity is reduced, sintering is likely to occur, resulting in that deactivation is likely to occur.

In the present invention, when platinum metal is loaded on the alumina carrier, the above-mentioned alumina carrier may be impregnated with a solution of platinum compound, dried, and then calcined at a predetermined temperature. As the platinum compound, chloride, bromide, ammonium salt, carbonyl compound, or various complex compounds, such as an amine complex, an ammine complex, and an acetylacetonato complex, of platinum can be mentioned. The platinum compound may be, for example, chloroplatinic acid, platinum acetylacetonate, ammonium platinate, bromo platinate, platinum dichloride, platinum tetrachloride hydrate, platinum carbonyl dichloride, dinitrodiamine platinate salt, or the like.

After the alumina carrier is impregnated with the above-mentioned solution of platinum compound, the alumina carrier to which the platinum compound adheres is dried at 50 to 200 °C for 0.5 to 48 hours, and thereafter is calcined at 350 to 600 °C for 0.5 to 48 hours, more preferably at 350 to 450 °C for 0.5 to 5 hours. Then, the calcined alumina carrier is subjected to hydrogen reduction treatment in a hydrogen gas atmosphere under a reduction condition at 350 to 600 °C for 0.5 to 48 hours, preferably at 350 to 550 °C for 3 to 24 hours. If the temperature during this hydrogen reduction is lower than 350 °C, there is a problem that platinum may not be sufficiently reduced, while if the temperature during the hydrogen reduction exceeds 600 °C, there is a problem that sintering of platinum particles occurs at the time of reduction and the metal dispersion degree decreases.

The method of producing the egg shell-type platinum-loaded alumina catalyst includes a step of impregnating the alumina carrier with an aqueous solution of compound containing one or more components selected from the group consisting of vanadium, chromium, molybdenum, and phosphorus, and thereafter performing drying and calcination, a step of loading platinum on the alumina carrier after calcination in an egg shell form, and a step of reducing the alumina carrier on which platinum is loaded in a hydrogen atmosphere.

In the cost reduction of dehydrogenation catalyst for the organic chemical hydride method / system, it is conceivable, next to extension of the catalyst life, to simplify the process by non-use of sulfur. The present inventors carried out extensive research on dehydrogenation catalyst which can achieve similar catalyst life without use of sulfur, and found that similar effect can be obtained by adding, instead of alkali metal or sulfur, a component selected from vanadium, chromium, molybdenum, and phosphorus as a second component other than platinum, thereby to complete the present invention.

Patent Document 1 discloses an egg shell-type platinum-loaded alumina catalyst in which platinum is loaded on an alumina carrier having a physical property of a surface area of 150 $m^2$/g or more, a pore volume of 0.40 $cm^3$/g or more, and an average pore diameter of 40 to 300 Å, with pores having a pore diameter in a range of $\pm$ 30 Å from the average pore diameter occupying 60 % or more of a total pore volume, such that 0.1 to 3 wt% of platinum calculated as elemental platinum is dispersed and loaded on an outer shell of the carrier cross section, as well as a catalyst obtained by adding alkali metal to this catalyst.

Namely, the present inventors found that by adding, instead of alkali metal, a component selected from vanadium, chromium, molybdenum, and phosphorus as a second component other than platinum to the egg shell-type platinum-loaded alumina catalyst disclosed in Patent Document 1, superior catalyst performance, particularly superior catalyst life, can be achieved compared to the alkali metal-added egg shell-type platinum-loaded alumina catalyst, thereby to complete the present invention.

The egg shell-type platinum-loaded alumina catalyst of the present invention is used, for example, as a dehydrogenation catalyst for a hydrogenated aromatic, which is utilized as a hydrogen energy carrier in the organic chemical hydride method, which is one of the methods for storing and transporting the hydrogen energy. The hydrogenated aromatic preferably is one member or a mixture of two or more members selected from the group consisting of a hydride of monocyclic aromatic, a hydride of bicyclic aromatic, and a hydride of compound having 3 or more aromatic rings. Also, the hydrogenated aromatic preferably is one member or a mixture of two or more members selected from the group consisting of methylcyclohexane, cyclohexane, dimethylcyclohexane, tetralin, decalin, methyldecalin, biphenyl, diphenylmethyl, dibenzotriol, and tetradecahydroanthracene.

When the platinum-loaded alumina catalyst is used in the dehydrogenation reaction of a hydrogenated aromatic such as methylcyclohexane in the organic chemical hydride method, catalyst deterioration is observed, in which the performance decreases gradually as the reaction time passes. The cause of the catalyst deterioration is carbon precipitation called coking. Coking is a phenomenon in which carbon precipitation occurs on the surface of platinum metal, which is an active metal mainly due to decomposition reaction of the raw material compound such as methylcyclohexane, and as a result, the effective active sites of the active metal are covered and do not function.

The catalytic reaction deterioration phenomenon is observed as a decrease of the conversion rate in the reaction test, and in the case of dehydrogenation reaction of a hydrogenated aromatic, it is known that the phenomenon is observed as a linear decrease of the conversion rate. Accordingly, by observing the change of the conversion rate over time in the reaction test, it is possible to evaluate relative superiority or inferiority of the catalyst life based on the inclination of the decrease. Further, since the conversion rate decreases linearly not only under actual reaction conditions but also in an accelerated reaction test for evaluating the catalyst life in a short time, it is possible to evaluate the catalyst life.

Hydrogen attracted attention as clean secondary energy from 1970s, and in Japan, research and development of hydrogen production technologies and fuel cells were promoted in the Sunshine Project from 1974 to 1992, the Moonlight Project from 1978 to 1992, and the New Sunshine Project from 1993 to 2001. Regarding large-scale storage and transportation technology of hydrogen, development of liquefied hydrogen method was started in the WE-NET project from 1992 to 2002. On the other hand, the history of development of the organic chemical hydride method is old, and goes back to the Euro-Quebec project which was carried out in 1980s as an international research and development project by Canada's Quebec government and twelve European countries. This plan proposed producing hydrogen by performing water electrolysis by using excess hydroelectric power abundantly present in Quebec and transporting the hydrogen across the Atlantic Ocean for use in Europe. As a hydrogen transportation method, discussion was made on the liquid hydrogen method as a first candidate, the liquid ammonia method as a second candidate, and the organic chemical hydride method as a third candidate. At that time, the organic chemical hydride method was called an MCH method. The Euro-Quebec project continued till about 1992 for approximately 10 years but the project ended with none of the methods being practically implemented, and since then, technology for large-scale hydrogen store and transport has not been practically implemented.

In Japan, development of the liquefied hydrogen method was promoted in WE-NET project implemented from 1992 to 2002, while the research of the organic chemical hydride method was promoted mainly by Japanese universities.

The applicant started the development of dehydrogenation catalyst in 2002 and made a first academic presentation in the World Hydrogen Energy Conference held in Yokohama in 2004, and from around this time, examples of research and development at companies were started to be released. At the present time, the large-scale hydrogen storage and transportation technologies for which research and development have been advanced to a demonstration level are only the liquefied hydrogen method and the organic chemical hydride method, which is proposed by the applicant.

The organic chemical hydride method (OCH method) is a method in which hydrogen is subjected to hydrogenation reaction with an aromatic such as toluene (TOL) and converted to a saturated cyclic compound such as methylcyclohexane (MCH) having the hydrogen taken in the molecule thereof so that "storage" and "transportation" are achieved in a liquid state under normal temperature and normal pressure and a necessary amount of hydrogen is taken out by dehydrogenation reaction and used at the place of use. Thus, the method includes a hydrogenation reaction (hydrogen storage reaction) for making hydrogen react with toluene and a dehydrogenation reaction (hydrogen generation reaction) for generating hydrogen from MCH and recovering toluene. TOL generated after taking out hydrogen is recovered and repeatedly used as a container (carrier) of hydrogen.

Since hydrogen is an explosive gas, there is a potentially high risk in storing and transporting hydrogen as it is in a large scale. In the present method, storage and transportation of hydrogen is performed with the hydrogen being retained in the molecule of MCH, which is a component of gasoline and diesel oil and is in the liquid state under normal temperature and normal pressure, and therefore, this method is highly safe in principle. Specifically, even if the tank and the reactor of the present system are caught in a fire, it would be similar to conventional oil refinery fires, and it is considered that the possibility of causing severe damage to the surrounding urban area is very low. The thought "accidents will eventually happen" is very important to safety measures, and that is why it is required to be safe in principle.

With this method, it is possible to store about 530 L of hydrogen gas in 1 L of liquid MCH. To physically decrease the volume of hydrogen gas to 1/500 or less, it is necessary to compress the hydrogen gas to 500 atm or higher or to cool the hydrogen gas to -253 °C or lower to make it liquid hydrogen which is 1/800 in volume, but according to the present method, by use of chemical reaction it is possible to decrease the volume to 1/500 under normal temperature and normal pressure. Also, since TOL and MCH are in the liquid state in a wide temperature range of -95 to 101 °C, they can be handled as a liquid such as water under any environment on the Earth. To establish a large-scale supply chain, it is necessary to procure hundreds of thousands of tons of TOL, but TOL is a fuel base material contained in high octane gasoline in a proportion of 10 wt% or more and also is a general purpose chemical product produced in 20 million tons per year worldwide to be used widely as industrial solvent. Therefore, TOL can be easily procured in large amounts.

From the foregoing, the primary feature of the present method is high safety that can lower the potential risk regarding large-scale storage and transportation of hydrogen to the level of the risk related to conventional gasoline storage and transportation in principle, and this is the first reason why the applicant focused on this method. Also, storage of TOL and MCH in large tanks and transportation of the same by chemical tankers and chemical lorries have been practically implemented from old times as chemical products. In the current trend of electrification of automobiles, demand for gasoline and diesel oil as automobile fuels is expected to decrease and the existing infrastructure therefor, such as storage tanks, could be diverted. This can be a significant merit.

Further, in a case where hydrogen is used in large scale as fuel for power generation in the future, it is expected that hydrogen fuel reserve will become necessary as the current oil reserve. TOL and MCH do not chemically change even if stored for a long period and in large scale, and there is no extra energy consumption or loss due to long-term storage, and therefore, by storing MCH in the tanks of current oil reserve base, it is possible to convert the oil reserve bae to a reserve base for hydrogen energy.

The applicant focused on the organic chemical hydride method which has the highest safety and is advantageous in cost because the existing infrastructure can be diverted, started the development of novel dehydrogenation catalyst, which is the key to practical implementation, in 2002, and succeeded first in the world in developing novel dehydrogenation catalyst that can be industrially applicable to the organic chemical hydride method. Thereafter, for the purpose of establishment of technology for the whole system, the applicant used the developed catalyst in the dehydrogenation process and combined it with the toluene hydrogenation process, which realizes hydrogen storage reaction, to construct a demonstration plant which continuously repeats the hydrogen storage and hydrogen generation at the same place in 2013. The demonstration operation was conducted from April 2013 to November 2014 for about 10, 000 hours in total, and it was confirmed that high performance as designed could be maintained stably, whereby the establishment of the technology was completed.

Thereafter, as the final stage of the development, a first-in-the-world demonstration of international hydrogen supply chain, in which about 200 tons of hydrogen will be actually transported from Brunei in Southeast Asia to Kawasaki waterfront in Japan by use of the present system, will be carried out as a project of NEDO (New Energy and Industrial Technology Development Organization) in 2020.

Japan has adopted a policy to promote practical implementation and dissemination of hydrogen energy as a national policy from the Fourth Strategic Energy Plan after the Great East Japan Earthquake, and following formulation of the Hydrogen Fuel Cell Technology Roadmap, the Hydrogen Basic Strategy was approved by the cabinet in 2017. Practical implementation of the aforementioned organic chemical hydride method is incorporated, as a hydrogen energy carrier for "storing" and "transporting" hydrogen energy in a large scale, in the Hydrogen Basic Strategy, which sets 30 ¥/Nm$^3$ as a target hydrogen supply price by 2030, and 20 ¥/Nm$^3$ by 2050. This requires cost reduction by continuous improvement technology development, and improvement of the catalyst performance is an important element of the cost reduction. Thus, the present invention is effective in the practical implementation of the organic chemical hydride method and highly useful industrially.

The egg shell-type platinum-loaded alumina catalyst not only can be used as a catalyst but also can be effectively used as an adsorbent or the like. The uniform-type platinum-loaded alumina catalyst is also useful as a filler of guard columns for preprocessing of adsorbing impurities and the like in the catalytic reaction process to which the catalyst of the present invention can be applied, as application to the organic chemical hydride method.

The egg shell-type platinum-loaded alumina catalyst according to the present embodiment may be used together with a uniform-type alkali-added platinum-loaded alumina catalyst. The uniform-type alkali-added platinum-loaded alumina catalyst preferably includes an alumina carrier, sulfur or a sulfur compound dispersed over an entire cross section of the alumina carrier, platinum dispersed and loaded over the entire cross section of the alumina carrier, and one or more alkali metals selected from the group consisting of sodium, potassium, and calcium. The alumina carrier preferably has a surface area of 150 m$^2$/g or more, a pore volume of 0.40 cm$^3$/g or more, and an average pore diameter of 40 to 300 Å, with pores having a pore diameter in a range of ± 30 Å from the average pore diameter occupying 60 % or more of a total pore volume. The amount of sulfur to be contained in the carrier is preferably 0.15 to 5 wt%, and more preferably 0.15 to 3.0 wt%, calculated as elemental sulfur. The most suitable sulfur content range is 0.15 to 3.0 wt. The loading amount of platinum is 0.05 to 5.0 wt%, preferably 0.1 to 3.0 wt%. The amount of alkali added is 0.1 to 5 wt%, preferably 0.3 to 3.0 wt%, and more preferably 0.5 to 1.5 wt%. The compound of the alkaline metal used when loading the alkaline metal to the uniform-type platinum-loaded alumina catalyst may be, for example, a chloride, bromide, iodide, nitrate, sulfate, acetate, propionic acid, and the like of the alkaline metal, which preferably is water soluble and/or soluble to an organic solvent such as acetone. Such a compound may be, for example, sodium chloride, sodium bromide, sodium iodide, sodium nitrate, sodium sulfate, sodium acetate, sodium propionate, potassium chloride, potassium bromide, potassium iodide, potassium nitrate, potassium sulfate, potassium acetate, potassium propionate, calcium chloride, calcium bromide, calcium iodide, calcium nitrate, calcium sulfate, calcium acetate, calcium propionate, or the like.

The egg shell-type platinum-loaded alumina catalyst and the uniform-type alkali-added platinum-loaded alumina catalyst are preferably charged in the same reactor. The egg shell-type platinum-loaded alumina catalyst and the uniform-type alkali-added platinum-loaded alumina catalyst may be arranged in series or may be arranged in a mutually mixed manner.

In this way, the egg shell-type platinum-loaded alumina catalyst can be favorably used in the dehydrogenation reaction of hydrogenated aromatic, such as methylcyclohexane, used as a hydrogen energy carrier, and contributes to practical implementation of the hydrogen storage and transportation system according to the organic chemical hydride method. Besides, there is a possibility that the uniform-type platinum-loaded alumina catalyst can be widely applied to the existing catalytic reaction processes in which the platinum-loaded alumina catalyst is used, and the industrial applicability is very high.

Hereinafter, preferable embodiments of the present invention will be specifically described based on Examples and Comparative Examples.

[Carrier Preparation]

A sodium aluminate aqueous solution was instantaneously added in hot dilute sulfuric acid while being vigorously stirred to obtain an aluminum hydroxide slurry suspension (pH10). This suspension was used as seed aluminum hydroxide and while stirring was continued, an operation of alternately adding the hot dilute sulfuric acid and the sodium aluminate aqueous solution at a constant interval was repeated to obtain filtered and washed cake. This cake was extruded and dried, and thereafter was calcined at 500 °C for 3 hours. The alumina carrier thus obtained had a BET surface area of 257 m$^2$/g, a pore volume as measured by a mercury injection method of 0.66 cm$^3$/g, an average pore diameter of 9.3 nm, and a sharp pore distribution in which most pores were concentrated around the average pore diameter. The volume occupied by pores with a pore diameter of 7-13 nm was 85 % or more of the total pore volume, and from this result, the obtained porous γ-alumina carrier satisfied the requirements of the surface area of 150 m$^2$/g or more, the pore volume of 0.40 cm$^3$/g or more, the average pore diameter of 40 to 300 Å, and the pores having a pore diameter in a range of ± 30 Å from the average pore diameter occupying 60 % or more of the total pore volume.

[Comparative Example 1 (catalyst No. 1)]

For the purpose of comparison with the Examples, an egg shell-type platinum-loaded alumina catalyst (catalyst No. 1) according to Comparative Example 1 was produced. 40 mL of chloroplatinic acid aqueous solution with a concentration of 0.026 mol/L, which was prepared so that the pH value was 2.0, was added to 20 g of the porous γ-alumina carrier prepared as mentioned above, and this was left for 3 hours for impregnation before water was removed with an evaporator. Subsequently, the resultant was dried for 3 hours at 120 °C and then was calcined for 3 hours at 450 °C in a muffle furnace under air flow, whereby an egg shell-type platinum-loaded alumina catalyst on which 1 wt% of platinum was loaded (catalyst No. 1) was prepared.

[Comparative Example 2 (catalyst No. 2)]

40 mL of sodium (Na) nitrate aqueous solution with a concentration of 0.085 mol/L was added to 20 g of the egg shell-type platinum-loaded alumina catalyst prepared and obtained similarly to Comparative Example 1, and this was left for 3 hours for impregnation before water was removed with an evaporator. Subsequently, the resultant was dried for 3 hours at 120 °C and then was calcined for 3 hours at 450 °C in muffle furnace under air flow, whereby, an egg shell-type alkali-added platinum-loaded alumina catalyst on which 0.8 wt% of sodium (Na) and 1 wt% of platinum were loaded (catalyst No. 2) was obtained. The egg shell-type alkali-added platinum-loaded alumina catalyst according to Comparative Example 2 corresponds to the catalyst described in the aforementioned Patent Document 1.

[Comparative Example 3 (catalyst No. 3)]

40 mL of ammonium sulfate aqueous solution with a concentration of 0.16 mol/L was added to 20 g of the porous γ-alumina carrier prepared according to the aforementioned method, and this was left for 3 hours for impregnation before water was removed with an evaporator. Subsequently, the resultant was dried for 3 hours at 120 °C and then was calcined for 3 hours at 500 °C in a muffle furnace under air flow, whereby an alumina carrier containing sulfur was obtained. 40 mL of chloroplatinic acid aqueous solution with a concentration of 0.026 mol/L, which was prepared so that pH was 2.0, was added to 20 g of the alumina carrier containing sulfur thus prepared, and after impregnation for making the loading amount of platinum after calcination be 1.0 wt%, moisture was removed with an evaporator. Subsequently, the resultant was dried for 3 hours at 120 °C and then was calcined for 3 hours at 450 °C in a muffle furnace under air flow, whereby a uniform-type sulfur-platinum-loaded alumina catalyst which contained 1.0 wt% of sulfur and on which 1.0 wt% of platinum was dispersed and loaded (catalyst No. 3) was obtained. This method conforms to the preparation method described in Example 3 of Patent Document 2.

[Comparative Example 4 (catalyst No. 4)]

20 g of alumina carrier containing sulfur prepared by the method same as in Comparative Example 3 was impregnated with 5.6 mL of dinitrodiamine platinum aqueous solution with a concentration of 0.18 mol/L so that the loading amount of platinum after calcination was 1.0 wt%, and thereafter, moisture was removed with an evaporator. Subsequently, the resultant was dried for 3 hours at 120 °C and then was calcined for 3 hours at 450 °C in a muffle furnace under air flow, whereby an egg shell-type sulfur-platinum-loaded alumina catalyst which contained 1.0 wt% of sulfur and on which 1.0 wt% of platinum was loaded in an egg shell manner was obtained. 40 mL of sodium nitrate (Na) aqueous solution with a concentration of 0.17 mol/L was added to 20 g of the egg shell-type sulfur-platinum-loaded alumina catalyst thus prepared, and this was left for 3 hours for impregnation before water was removed with an evaporator. Subsequently, the resultant was dried for 3 hours at 120 °C and then was calcined for 3 hours at 450 °C in a muffle furnace under air flow, whereby an egg shell-type alkali-added sulfur-platinum-loaded alumina catalyst on which 0.8 wt% of sodium (Na) and 1 wt% of platinum were loaded (catalyst No. 4) was obtained.

[Example 1 (catalyst No. 5)]

20g of the porous γ-alumina carrier prepared by the method described in "Carrier Preparation" is impregnated with 33.2 mL of an aqueous solution obtained by adding oxalic acid to an ammonium metavanadate solution with a concentration of 0.12 mol/L in the same weight and dissolving oxalic acid therein, and this was left for 3 hours before water was removed with an evaporator. Subsequently, the resultant was dried for 3 hours at 120 °C and then was calcined for 3 hours at 500 °C in a muffle furnace under air flow, whereby an alumina carrier containing 1.2 wt% of vanadium (V) as the second component was obtained. Platinum was loaded on the vanadium-containing alumina carrier thus prepared by a method similar to Comparative Example 4 (catalyst No.4) using dinitrodiamine platinum aqueous solution, whereby an egg shell-type vanadium-added platinum-loaded alumina catalyst on which 1.2 wt% of vanadium and 1.0 wt% of platinum were loaded (catalyst No.5) was obtained.

[Example 2 (catalyst No.6)]

20 of the porous γ-alumina carrier prepared by the method described in "Carrier Preparation" was impregnated with 32.0 mL of chromium nitrate aqueous solution with a concentration of 0.12mol/L, and this was left for 3 hours before water was removed with an evaporator. Subsequently, the resultant was dried for 3 hours at 120 °C and then was calcined for 3 hours at 500 °C in a muffle furnace under air flow, whereby an alumina carrier containing 1.2 wt% of chromium (Cr) as the second component was obtained. Platinum was loaded on the chromium-containing alumina carrier thus prepared by a method similar to Comparative Example 4 (catalyst No.4) using dinitrodiamine platinum aqueous solution, whereby an egg shell-type chromium-added platinum-loaded alumina catalyst on which 1.2 wt% of chromium and 1.0 wt% of platinum were loaded (catalyst No.6) was obtained.

[Example 3 (catalyst No.7)]

20 of the porous γ-alumina carrier prepared by the method described in "Carrier Preparation" is impregnated with 32.0 mL of ammonium molybdate aqueous solution with a concentration of 0.009 mol/L, and this was left for 3 hours before water was removed with an evaporator. Subsequently, the resultant was dried for 3 hours at 120 °C and then was calcined for 3 hours at 500 °C in a muffle furnace under air flow, whereby an alumina carrier containing 1.3 wt% of molybdenum (Mo) as the second component was obtained. Platinum was loaded on the molybdenum-containing alumina carrier thus prepared by a method similar to Comparative Example 4 (catalyst No.4) using dinitrodiamine platinum aqueous solution, whereby an egg shell-type molybdenum-added platinum-loaded alumina catalyst on which 1.3 wt% of molybdenum and 1.0 wt% of platinum were loaded (catalyst No.7) was obtained.

[Example 4(catalyst No.8)]

20 of the porous γ-alumina carrier prepared by the method described in "Carrier Preparation" was impregnated with 32.0 mL of ammonium dihydrogen phosphate aqueous solution with a concentration of 0.20 mol/L, and this was left for 3 hours before water was removed with an evaporator. Subsequently, the resultant was dried for 3 hours at 120 °C and then was calcined for 3 hours at 500 °C in a muffle furnace under air flow, whereby an alumina carrier containing 0.9 wt% of phosphorus (P) as the second component was obtained. Platinum was loaded on the phosphorus-containing alumina carrier thus prepared by a method similar to Comparative Example 4 (catalyst No.4) using dinitrodiamine platinum aqueous solution, whereby an egg shell-type phosphorus-added platinum-loaded alumina catalyst on which 0.9 wt% of phosphorus and 1.0 wt% of platinum were loaded (catalyst No.8) was obtained.

[Examples 5 and 6 (catalyst Nos. 9 and 10)]

By a method similar to Example 1 with varied concentrations of ammonium metavanadate aqueous solution, alumina carriers respectively containing 0.5 wt% and 2.0 wt% of vanadium (V) as the second component were prepared, and thereafter, platinum was loaded thereon in a similar way, whereby an egg shell-type vanadium-added platinum-loaded alumina catalyst on which 0.5 wt% of vanadium and 1.0 wt% of platinum were loaded (catalyst No.9) and an egg shell-type vanadium-added platinum-loaded alumina catalyst on which 2.0 wt% of vanadium and 1.0 wt% of platinum were loaded (catalyst No.10) were obtained.

[Example 7 (catalyst No.11)]

By a method similar to Example 1 with a varied concentration of ammonium metavanadate aqueous solution, an alumina carrier containing 0.5 wt% of vanadium (V) as the second component was prepared, and thereafter, the concentration of dinitrodiamine platinum aqueous solution was adjusted in a similar way, whereby an egg shell-type vanadium-added platinum-loaded alumina catalyst on which 0.5 wt% of vanadium and 0.5 wt% of platinum were loaded (catalyst No.11) was obtained.

[Examples 8-10 (catalyst Nos. 12-14)]

By a method similar to Example 2 with varied concentrations of chromium nitrate aqueous solution, alumina carriers respectively containing 0.5 wt%, 2.0 wt%, and 3.0 wt% of chromium (Cr) as the second component were prepared, and thereafter, platinum was loaded thereon in a similar way, whereby an egg shell-type chromium-added platinum-loaded alumina catalyst on which 0.5 wt% of chromium and 1.0 wt% of platinum were loaded (catalyst No.12), an egg shell-type chromium-added platinum-loaded alumina catalyst on which 2.0 wt% of chromium and 1.0 wt% of platinum were loaded (catalyst No.13), and an egg shell-type chromium-added platinum-loaded alumina catalyst on which 3.0 wt% of chromium and 1.0 wt% of platinum were loaded (catalyst No.14) were obtained.

[Examples 11 and 12 (catalyst Nos. 15 and 16)]

By a method similar to Example 3 with varied concentrations of ammonium molybdate aqueous solution, alumina carriers respectively containing 0.5 wt% and 2.0 wt% of molybdenum (Mo) as the second component were prepared, and thereafter, platinum was loaded thereon in a similar way, whereby an egg shell-type molybdenum-added platinum-loaded alumina catalyst on which 0.5 wt% of molybdenum and 1.0 wt% of platinum were loaded (catalyst No.15) and an egg shell-type molybdenum-added platinum-loaded alumina catalyst on which 2.0 wt% of molybdenum and 1.0 wt% of platinum were loaded (catalyst No.16) were obtained.

(Accelerated Reaction Test)

Next, a reaction test was performed using the aforementioned Comparative Examples 1 to 4 (catalyst Nos. 1 to 4) and Examples 1 to 12 (catalyst Nos. 5 to 16). The reaction test was performed under accelerated condition so that differences in performance were obtained in a relatively short time. 1.3 g of each catalyst was diluted with 11.7 g of $\alpha$-alumina beads and was charged in a lengthwise central part of a stainless steel reaction tube which had an inside diameter of 12.6 mm and a length of 300 mm and which was equipped with a protective tube for a thermocouple whose outer dimension was 1/8 inch in the center of the cross section of the reaction tube. Spherical $\alpha$-alumina beads with a diameter of 1 mm were placed on the upper side of the catalyst as a preheating layer. Thereafter, the temperature of the catalyst layer was raised to 380 °C, and pre-treatment reduction was carried out for 15 hours in a hydrogen stream. After the pre-treatment reduction finished, the catalyst layer inlet temperature was set to 400 °C, the catalyst layer outlet temperature was set to 410 °C, the pressure was set to 0.3 MPa, the hydrogen gas supplied for the pre-treatment reduction was stopped, and methylcyclohexane (MCH) gas evaporated by a heater was supplied to the reactor at a flow rate of 2.9 L/h (LHSV: 8 h$^{-1}$). A cooler for cooling the gas exiting from the reactor was provided at the outlet of the reaction tube, and the resultant was collected in a stainless steel container and was separated into a liquid product such as liquefied toluene (TOL) and gas such as hydrogen gas. Composition analysis was performed separately for the collected liquid product and gas by gas chromatography, and the MCH conversion rate (%) and the TOL selectivity (%) from the reaction start to 140 hours later were calculated. Also, the catalyst after the reaction test was taken out, and an amount of carbon production, which is a cause for catalyst deterioration, was measured with a carbon/sulfur analyzer.

(Catalyst Life Test)

Next, by using the aforementioned Comparative Example 3 (catalyst No. 3) and Example 1 (catalyst No. 5), a long time reaction test was carried out as follows. 8.5 g of each catalyst was diluted with 129 g of spherical $\alpha$-alumina beads with an outer diameter of 5 mm and charged in a lengthwise central part of a stainless steel reaction tube which had an inside diameter of 21.2 mm and a length of 816 mm and which was equipped with a protection tube for a thermocouple whose outer dimension was 1/4 inch in the center of the cross section of the reaction tube. Spherical $\alpha$-alumina beads with an outer diameter of 1 mm were placed on the upper side of the catalyst as a preheating layer. The temperature of the catalyst layer was set to 380 °C, and pre-treatment reduction was carried out for 15 hours in a hydrogen stream. After the pre-treatment reduction finished, the catalyst layer inlet temperature was set to 280 °C, the catalyst layer outlet temperature was set to 380 °C, the reaction pressure was set to 0.55 MPa, and MCH gas was supplied to the reactor at a flow rate of 2.8L/h (LHSV: 2.6 h$^{-1}$). A cooler for cooling the gas exiting from the reactor was provided at the outlet of the reaction tube, and the resultant was collected in a stainless steel container and was separated into a liquid product such as liquefied toluene and gas such as hydrogen gas. Composition analysis was performed separately for the collected liquid product and gas by gas chromatography, and the MCH conversion rate (%) and the TOL selectivity (%) from the reaction start to 1400 hours later were calculated.

The result of the aforementioned accelerated reaction test is shown in Tables 1 to 4, and the result of the catalyst life test is shown in Table 5.

Table 1 accelerated test comparison between second component elements

| catalyst No. | | second component | | Pt | after 20 hours | | after 140 hours | | amount of carbon production |
|---|---|---|---|---|---|---|---|---|---|
| | | element | loading amount | | MCH conversion rate | TOL selectivity | MCH conversion rate | TOL selectivity | |
| | | | (wt%) | (wt%) | (%) | (%) | (%) | (%) | (wt%) |
| 1 | Comparative Example 1 | none | - | 1.0 | 95.7 | 99.3 | 90.4 | 99.6 | 2.6 |
| 2 | Comparative Example 2 | Na | 0.8 | 1.0 | 94.5 | 99.6 | 88.0 | 99.8 | 1.5 |
| 3 | Comparative Example 3 | S | 1.0 | 1.0 | 98.7 | 99.8 | 95.0 | 99.8 | 2.6 |
| 4 | Comparative Example 4 (ES-Na) | S-Na | 1.0 - 0.8 | 1.0 | 97.3 | 98.0 | 93.9 | 97.9 | 0.5 |
| 5 | Example 1 | V | 1.2 | 1.0 | 99.5 | 99.9 | 98.4 | 99.9 | 0.7 |
| 6 | Example 2 | Cr | 1.2 | 1.0 | 99.4 | 99.9 | 98.1 | 99.9 | 1.4 |
| 7 | Example 3 | Mo | 1.3 | 1.0 | 98.5 | 99.9 | 96.2 | 99.9 | 0.8 |
| 8 | Example 4 | P | 0.9 | 1.0 | 98.8 | 99.7 | 97.0 | 99.9 | 1.2 |

From Table 1, it can be seen that compared to the egg shell-type platinum-loaded alumina catalyst of Comparative Example 1 (catalyst No. 1) in which platinum was dispersed and loaded on only the outer shell part of the carrier and the egg shell-type alkali-added platinum-loaded alumina catalyst of Comparative Example 2 (catalyst No. 2), the uniform-type sulfur-platinum-loaded alumina catalyst of Comparative Example 3 (catalyst No. 3) in which sulfur was dispersed and loaded over the entirety of the alumina carrier and platinum was dispersed and loaded over the entirety of the alumina carrier demonstrated a high MCH conversion rate and a high TOL selectivity both at an initial stage of the accelerated reaction test and after 140 hours of reaction (the MCH conversion rate was 98.7 % and 95.0 %, respectively), and thus, is excellent in the catalyst stability and selectivity.

As described above, the uniform-type sulfur-platinum-loaded alumina catalyst of Comparative Example 3 demonstrates excellent catalyst performance, but the amount of carbon production after the reaction test was 2.6 wt%, which is greater than that of the egg shell-type alkali-added platinum-loaded alumina catalyst of Comparative Example 2 (catalyst No. 2). From this result, it is inferred that in the catalyst of Comparative Example 3, dispersity is improved due to uniform dispersion of platinum, which is the catalyst component, over the carrier, and this provides numerous active sites effective for reaction, whereby even though the amount of carbon precipitation is increased, active sites effective for reaction are still held.

Similarly, from Table 1, it can be seen that in each of Examples 1 to 4 (Example 1: vanadium, Example 2: chromium, Example 3: molybdenum, Example 4: phosphorus ), the MCH conversion rate 20 hours after the reaction start was equivalent to or higher than that of Comparative Example 3, and the MCH conversion rate 140 hours after was maintained to a value equal to or higher than that of Comparative Example 3, and thus, the catalyst was better in stability compared to Comparative Example 3. Also, the TOL selectivity was 99.9 %, which was better than that of Comparative Example 3, and the amount of carbon production after the reaction test was 0.7 to 1.4 %, which was about 1/3 to 1/2 from 2.6 wt% of Comparative Example 3. The improvement of TOL selectivity was 0.1 % numerically, but since the amount of impurities produced is calculated according to the formula below, the improvement of the TOL selectivity from 99.8 % to 99.9 % suppresses the amount of impurities produced to about a half.

$$\text{Amount of impurities produced} = \text{Supply amount of MCH} \times \text{MCH conversion rate} /100$$

$$\times (100 - \text{TOL selectivity}) /100$$

The egg shell-type platinum-loaded alumina catalysts of Examples 1 to 4 exhibited a high MCH conversion rate and a high TOL selectivity even compared to the egg shell-type platinum-loaded alumina catalyst of Comparative Example 4 in which sulfur and sodium were added. From these, it can be seen that when the additive to the egg shell-type platinum-loaded alumina catalyst is vanadium, chromium, molybdenum, or phosphorus, the MCH conversion rate and the TOL selectivity are improved better than when the additive is sodium, which is an alkali metal.

Table 2 accelerated test effect of the loading amount of Vd, reduction of amount of Pt

| catalyst No. | | second component | | Pt | after 20 hours | | after 140 hours | | amount of carbon production |
|---|---|---|---|---|---|---|---|---|---|
| | | element | loading amount | | MCH conversion rate | TOL selectivity | MCH conversion rate | TOL selectivity | |
| | | | (wt%) | (wt%) | (%) | (%) | (%) | (%) | (wt%) |
| 5 | Example 1 | V | 1.2 | 1.0 | 99.5 | 99.9 | 98.4 | 99.9 | 0.7 |
| 9 | Example 5 | V | 0.5 | 1.0 | 99.4 | 99.9 | 98.2 | 99.9 | 0.8 |
| 10 | Example 6 | V | 2.0 | 1.0 | 96.9 | 99.9 | 96.6 | 99.9 | 0.5 |
| 11 | Example 7 | V | 0.5 | 0.5 | 99.5 | 99.9 | 97.0 | 99.9 | 0.5 |

From Table 2 for assessing the effect of the loading amount of vanadium, it can be seen that when the loading amount of vanadium is increased to about 2.0 wt% (Example 6), the MCH conversion rate starts to decrease. On the other hand, regarding the amount of carbon production, the results show that there is no significant influence so long as the loading amount of vanadium is 0.5 wt% (Example 5) or greater.

Also, from Table 2, it can be seen that in Example 7 in which the weight ratio of vanadium and platinum was maintained to 1: 1 and the amount of platinum was reduced to 0.5 wt%, the MCH conversion rates after 20 hours and after 140 hours were higher than those of Comparative Example 3 (see Table 1). This indicates that by adopting an egg shell-type platinum-loaded alumina catalyst to which the second metal component is added, it is possible to increase the amount of platinum present on the outer periphery of the catalyst which is effective for the reaction and thereby to decrease the loading amount of expensive platinum.

Table 3 accelerated test effect of the loading amount of Cr

| catalyst No. | | second component | | Pt | after 20 hours | | after 140 hours | | amount of carbon production |
| | | element | loading amount | | MCH conversion rate | TOL selectivity | MCH conversion rate | TOL selectivity | |
| | | | (wt%) | (wt%) | (%) | (%) | (%) | (%) | (wt%) |
|---|---|---|---|---|---|---|---|---|---|
| 6 | Example 2 | Cr | 1.2 | 1.0 | 99.4 | 99.9 | 98.1 | 99.9 | 1.4 |
| 12 | Example 8 | Cr | 0.5 | 1.0 | 97.7 | 99.8 | 95.5 | 99.8 | 1.2 |
| 13 | Example 9 | Cr | 2.0 | 1.0 | 98.7 | 99.9 | 97.0 | 99.9 | 1.0 |
| 14 | Example 10 | Cr | 3.0 | 1.0 | 96.0 | 99.9 | 95.1 | 99.9 | 0.8 |

From Table 3 for assessing the effect of the loading amount of chromium, it can be seen that when the loading amount of chromium is increased to about 3.0 wt%, the MCH conversion rate starts to decrease. In the case of chromium, with the loading amount of 0.5 wt%, the TOL selectivity is approximately equivalent to that of Comparative Example 3, and the loading to 1.0 wt% or greater is effective for increasing the selectivity. On the other hand, regarding the amount of carbon production, the results show that there is no significant influence so long as the loading amount of chromium is 0.5 wt% or greater.

Table 4 accelerated test effect of the loading amount of Mo

| catalyst No. | | second component | | Pt | after 20 hours | | after 140 hours | | amount of carbon production |
| | | element | loading amount | | MCH conversion rate | TOL selectivity | MCH conversion rate | TOL selectivity | |
| | | | (wt%) | (wt%) | (%) | (%) | (%) | (%) | (wt%) |
|---|---|---|---|---|---|---|---|---|---|
| 7 | Example 3 | Mo | 1.3 | 1.0 | 98.5 | 99.9 | 96.2 | 99.9 | 0.8 |
| 15 | Example 11 | Mo | 0.5 | 1.0 | 97.7 | 99.9 | 94.5 | 99.9 | 0.8 |
| 16 | Example 12 | Mo | 2.0 | 1.0 | 97.2 | 99.9 | 90.4 | 99.8 | 0.7 |

From Table 4 for assessing the effect of the loading amount of molybdenum, it can be seen that in the cases where the loading amount of molybdenum was 0.5 wt% and 2.0 wt%, the MCH conversion rate was slightly low compared to the case where the loading amount of molybdenum was 1.3 wt%, and that the most favorable MCH conversion rate and stability were demonstrated in the case where, the loading amount of molybdenum was 1.0 wt% or greater.

Table 5 shows a result of the catalyst life test performed on Example 1 (catalyst No. 5, vanadium 1.8 wt%), which from the accelerated test results of Examples 1 to 12, demonstrated an MCH conversion rate that was relatively high and changed stably and an amount of carbon production that was relatively small, and Comparative Example 3.

Table 5 life test comparison between second component elements

| catalyst No. | | second component | | Pt | after 50 hours | | after 1200 hours | | amount of carbon production |
| | | element | loading amount | | MCH conversion rate | TOL selectivity | MCH conversion rate | TOL selectivity | |
| | | | (wt%) | (wt%) | (%) | (%) | (%) | (%) | (wt%) |
| 3 | Comparative Example 3 | S | 1.0 | 1.0 | 97.5 | 99.6 | 96.7 | 99.7 | 2.7 |
| 5 | Example 1 | V | 1.2 | 1.0 | 97.1 | 99.9 | 96.7 | 99.9 | 1.1 |

From Table 5, in the egg shell-type platinum-loaded alumina catalyst of Example 1 (catalyst No.5) to which the second component was added, the MCH conversion rate decreased by only 0.4 % over the period from 50 hours after the reaction start to 1200 hours after the reaction start, while in the uniform-type platinum-loaded alumina catalyst of Comparative Example 3 (catalyst No.3), the MCH conversion rate decreased by 0.8 %, and thus, the egg shell-type second component-added platinum-loaded alumina catalyst is expected to have a long catalyst life. Also, regarding the TOL selectivity, the egg shell-type second component-added platinum-loaded alumina catalyst maintained 99.9 % over the period from 50 hours after the reaction start to 1200 hours after the reaction start, which is higher than the TOL selectivity of the uniform-type platinum-loaded alumina catalyst of Comparative Example 3 (catalyst No.3), and thus, it can be seen that the egg shell-type second component-added platinum-loaded alumina catalyst is a catalyst that produces a small amount of impurities. In addition, the amount of carbon production after 1200 hours of reaction test was 1.1 % in Example 1, which is smaller than 2.7% in Comparative Example 3, and thus, it is indicated that by suppressing the amount of carbon production, an effect of enhancing the stability of the catalyst can be obtained.

INDUSTRIAL APPLICABILITY

The egg shell-type platinum-loaded alumina catalyst according to the present invention can be favorably used in the dehydrogenation reaction of hydrogenated aromatics such as methylcyclohexane used as a hydrogen energy carrier, and can contribute to practical implementation of the hydrogen storage and transportation system by organic chemical hydride method. Besides, there is a possibility that the alkali-added uniform-type platinum-loaded alumina catalyst according to the present invention can be widely applied to existing catalytic reaction processes in which the platinum-loaded alumina catalyst is used. Thus, the present invention has very high industrial applicability.

LIST OF REFERENCE NUMERALS

1    carrier
2    metal loading part

**Claims**

**1.** An egg shell-type platinum-loaded alumina catalyst, comprising:

an alumina carrier;
platinum dispersed and loaded on an outer shell of the alumina carrier; and
one or more second components selected from the group consisting of vanadium, chromium, molybdenum, and phosphorus.

**2.** The egg shell-type platinum-loaded alumina catalyst according to claim 1, wherein a content of the platinum is 0.05 to 5.0 wt% calculated as elemental platinum.

**3.** The egg shell-type platinum-loaded alumina catalyst according to claim 1 or 2, wherein a content of each second component is 0.1 to 5.0 wt% calculated as each element.

**4.** The egg shell-type platinum-loaded alumina catalyst according to any one of claims 1 to 3, wherein the alumina carrier has a surface area of 150 m$^2$/g or more, a pore volume of 0.40 cm$^3$/g or more, and an average pore diameter of 40 to 300 Å, with pores having a pore diameter in a range of $\pm$ 30 Å from the average pore diameter occupying 60 % or more of a total pore volume.

**5.** A method of producing an egg shell-type platinum-loaded alumina catalyst, comprising:

a step of impregnating an alumina carrier with an aqueous solution of compound containing one or more components selected from the group consisting of vanadium, chromium, molybdenum, and phosphorus, and thereafter performing drying and calcination;
a step of loading platinum on the alumina carrier after calcination in an egg shell form; and
a step of reducing the alumina carrier on which platinum is loaded in a hydrogen atmosphere.

6. A method of dehydrogenating a hydrogenated aromatic, comprising dehydrogenating a hydrogenated aromatic by using the egg shell-type platinum-loaded alumina catalyst according to any one of claims 1 to 4.

7. The method of dehydrogenating a hydrogenated aromatic according to claim 6, wherein the hydrogenated aromatic is one member or a mixture of two or more members selected from the group consisting of a hydride of monocyclic aromatic, a hydride of bicyclic aromatic, and a hydride of compound having 3 or more aromatic rings.

8. The method of dehydrogenating a hydrogenated aromatic according to claim 4, wherein the hydrogenated aromatic is one member or a mixture of two or more members selected from the group consisting of methylcyclohexane, cyclohexane, dimethylcyclohexane, tetralin, decalin, methyldecalin, biphenyl, diphenylmethyl, dibenzotriol, and tetradecahydroanthracene.

9. The method of dehydrogenating a hydrogenated aromatic according to any one of claims 4 to 6, which uses both of the egg shell-type platinum-loaded alumina catalyst and a uniform-type platinum-loaded alumina catalyst.

# Fig.1

(A)                    (B)

## INTERNATIONAL SEARCH REPORT

| International application No. |
| --- |
| PCT/JP2020/017564 |

A. CLASSIFICATION OF SUBJECT MATTER
B01J 23/648(2006.01)i; B01J 23/652(2006.01)i; B01J 27/185(2006.01)i; C01B
3/26(2006.01)i; C07C 5/367(2006.01)i
FI: B01J23/648 M; B01J23/652 M; B01J27/185 M; C01B3/26; C07C5/367
According to International Patent Classification (IPC) or to both national classification and IPC

B. FIELDS SEARCHED

Minimum documentation searched (classification system followed by classification symbols)
B01J21/00-38/74; C01B3/26; C07C5/367

Documentation searched other than minimum documentation to the extent that such documents are included in the fields searched
Published examined utility model applications of Japan         1922-1996
Published unexamined utility model applications of Japan        1971-2020
Registered utility model specifications of Japan               1996-2020
Published registered utility model applications of Japan       1994-2020

Electronic data base consulted during the international search (name of data base and, where practicable, search terms used)
JSTPlus/JST7580/JSTChina (JDreamIII)

C. DOCUMENTS CONSIDERED TO BE RELEVANT

| Category* | Citation of document, with indication, where appropriate, of the relevant passages | Relevant to claim No. |
| --- | --- | --- |
| X | JP 60-82137 A (MITSUBISHI HEAVY INDUSTRIES, LTD.) 10 May 1985 (1985-05-10) claims, page 2, lower left column, lines 10-19, page 2, lower right column, lines 6-8, example 3, table 4 | 1-3, 5 |
| X | JP 58-177146 A (TOYOTA MOTOR CORP.) 17 October 1983 (1983-10-17) claims, example 1 | 1-3, 5 |
| X | JP 2005-525933 A (SHELL INTERNATIONALE RESEARCH MAATSCHAPPIJ B.V.) 02 September 2005 (2005-09-02) claims 1-3, 9, 12, examples 1, 6, 11-14 | 1-3 |
| Y | JP 4652695 B2 (CHIYODA CORPORATION) 16 March 2011 (2011-03-16) claims, paragraphs [0050]-[0066] | 1-9 |
| Y | JP 2005-138024 A (SEKISUI CHEMICAL CO., LTD.) 02 June 2005 (2005-06-02) claims 7, 8, paragraphs [0001], [0022], [0038], [0043], [0047], [0069], [0072] | 1-9 |

☒ Further documents are listed in the continuation of Box C.       ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 June 2020 (15.06.2020) | 30 June 2020 (30.06.2020) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2020/017564

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2001-198469 A (SEKISUI CHEMICAL CO., LTD.) 24 July 2001 (2001-07-24) claims, paragraphs [0026]-[0032], [0074]-[0075], [0085] | 1-4, 6-9 |
| Y | JP 2008-273778 A (ICHIKAWA, Masaru) 13 November 2008 (2008-11-13) claims, paragraphs [0060]-[0064] | 1-9 |
| Y | JP 4142733 B2 (CHIYODA CORPORATION) 03 September 2008 (2008-09-03) claims, examples 1, 6 | 9 |

Form PCT/ISA/210 (continuation of second sheet) (January 2015)

INTERNATIONAL SEARCH REPORT
Information on patent family members

International application No.

PCT/JP2020/017564

| Patent Documents referred in the Report | Publication Date | Patent Family | Publication Date |
|---|---|---|---|
| JP 60-82137 A | 10 May 1985 | (Family: none) | |
| JP 58-177146 A | 17 Oct. 1983 | (Family: none) | |
| JP 2005-525933 A | 02 Sep. 2005 | WO 2003/099433 A1 claims 1-3, 9, 12, examples 1, 6, 11-14 US 2003/0220193 A1 EP 1507586 A1 CN 1655867 A KR 10-2004-0111662 A | |
| JP 4652695 B2 | 16 Mar. 2011 | (Family: none) | |
| JP 2005-138024 A | 02 Jun. 2005 | (Family: none) | |
| JP 2001-198469 A | 24 Jul. 2001 | (Family: none) | |
| JP 2008-273778 A | 13 Nov. 2008 | WO 2008/136264 A1 CN 101801841 A | |
| JP 4142733 B2 | 03 Sep. 2008 | US 2009/0105511 A1 claims, examples 1, 6 WO 2006/137358 A1 EP 1894626 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4652695 B

- JP 4142733 B

**Non-patent literature cited in the description**

- **OKADA YOSHIMI.** *Energy/Natural Resources,* 2018, vol. 33 (3), 168
- **OKADA YOSHIMI.** *Bulletin of The High Pressure Gas Safety Institute of TOKYO,* August 2019

- *Agency for Natural Resources and Energy, Hydrogen Basic Strategy,* December 2017